Europäisches Patentamt

European Patent Office

Office européen des brevets

(11) Publication number: **0 540 751 A1**

# EUROPEAN PATENT APPLICATION
## published in accordance with Art. 158(3) EPC

(21) Application number: 92910149.1

(22) Date of filing: **18.05.92**

(86) International application number:
**PCT/JP92/00638**

(87) International publication number:
**WO 92/20699 (26.11.92 92/29)**

(51) Int. Cl.5: **C07J 17/00**

(30) Priority: **20.05.91 JP 142723/91**

(43) Date of publication of application:
**12.05.93 Bulletin 93/19**

(84) Designated Contracting States:
**AT BE CH DE DK ES FR GB GR IT LI LU MC NL SE**

(71) Applicant: **Eisai Co., Ltd.**
**6-10, Koishikawa 4-chome Bunkyo-ku Tokyo 112(JP)**

(72) Inventor: **TSUDA, Toshiro**
**4-5-94, Ninomiya**
**Tsukuba-shi, Ibaraki 305(JP)**
Inventor: **WATANABE, Sumio**
**14-2, Nakayoshiike, Saito, Fuso-machi Niwa-gun, Aichi 480-01(JP)**

(74) Representative: **Hansen, Bernd, Dr.**
**Dipl.-Chem. et al**
**Hoffmann, Eitle & Partner Patent- und Rechtsanwälte Arabellastrasse 4 Postfach 81 04 20**
**W-8000 München 81 (DE)**

(54) STEROID COMPOUND COMBINED WITH POLYSACCHARIDE.

(57) A compound which can maintain a blood steroid level unchanged for long after administration, obtained by combining a steroid derivative with a polysaccharide directly or via a spacer. Desirable examples of the steroid derivative include ones having carboxyl and/or hydroxyl groups, and those of the spacer include polybasic carboxylic acids, while those of the polysaccharide include water-soluble ones such as dextran. When administered, the compound releases the steroid in the blood and the steroid level is kept constant for long.

Industrially Applicable Field

The present invention relates to a compound prepared by combining chemically a steroid derivative with a polysaccharide.

Prior Art

Since steroids have in general very low solubility in water, such a steroid is often allowed to be soluble by the use of a surfactant when it is intended to use the steroid as an injection or the like. On one hand, the present inventors have specially developed a technique by which continuous effect of medicine is afforded to such medicine which disappears rapidly in the body by means of combining a medicine with a polysaccharide (Japanese Patent Application Laid−open No. 220201/91).

Problems to be Solved by the Invention

Recently, such side effects derived from use of surfactants for solubilizing slightly soluble medicines such as steroids and the like become problems in case of injecting such medicines.

Furthermore, there is such a fear that when steroids are intravenously administered, a concentration of the steroid administered in blood becomes extremely high immediately after the administration thereof resulting in side effects. On the other hand, the concentration of such steroid administered in blood decreases rapidly after the administration, and the steroid comes to lose the pharmaceutical efficiency, so that it is required to increase number of times in administration of the steroid, or drip intravenously the same.

The present inventors have studied energetically for solving the problems as described above. As a result, the inventors have found that these problems can be solved by the following means so that the present invention has been completed.

Means for Solving the Problems

More specifically, the present invention relates to a compound prepared by either combining directly a steroid derivative with a polysaccharide or combining them with each other by the use of a spacer.

The steroids used in the present invention are necessary for having carboxyl or hydroxyl groups in order to combine with polysaccharides or spacers. Sufficient is to present one equivalent or more of carboxyl or hydroxyl groups per one molecule of a steroid derivative, and in this case either carboxyl or hydroxyl groups may be present singly or both of them. An example of steroid derivatives includes hydrocortisone, prednisolone, dexamethasone, triamcinolone, aldosterone, corticosterone, cloprednol, deflazacort, prednisone, methylprednisolone, betamethasone, paramethasone, $9\alpha$−fluorocortisol, and deriva− tives thereof.

An example of the polysaccharides used in the present invention includes dextran, dextrin, starch, partially hydrolyzed starch, pullulan and the like, and a particularly preferred example includes dextran. A molecular weight of polysaccharide to be used is not specifically limited, but such molecular weight exhibiting desirable movement in vivo may be suitably selected. For example, as to dextran, an example of molecular weight thereof includes Dextran T−10 having molecular weight 10000, Dextran T−40 having molecular weight 40000, Dextran T−70 having molecular weight 70000, Dextran T−500 having molecular weight 500000 and the like. Preferred molecular weight is within a range of from 1,000 to 500,000, and more preferred molecular weight ranges from 10,000 to 200,000.

The term "spacer" used in the present invention means the residue of polycarboxylic acid which is ester−linked to both of steroid derivative and polysaccharide. An example of the spacer includes a residue obtained after ester linkage of both of a steroid derivative and a polysaccharide with each of the following polycarboxylic acids such as hymic acid, methylnadic acid, maleic acid, bromomaleic acid, dichloromaleic acid, mithraconic acid, dimethylmaleic acid, succinic acid, s−acetylmercaptosuccinic acid, methylsuccinic acid, methylenesuccinic acid, pyridinedicarboxylic acid, tetrahydrophthalic acid, phthalic acid, nitrophthalic acid, cyclohexanedicarboxylic acid, hexahydromethylphthalic acid, diglycolic acid, tetramethyleneglutaric acid, glutaric acid, phenylglutaric acid, dimethylglutaric acid, methylglutaric acid, ethylmethylglutaric acid, isatoic acid, homophthalic acid, micropentenedicarboxylic acid, methylcyclohexenedicarboxylic acid, phenylmaleic acid, cyclobutanedicarboxylic acid, dimethylsuccinic acid, methyltetrahydrophthalic acid, dibromomaleic acid, cyclopentanedicarboxylic acid, cyclohexanediacetic acid, oxauracil, and thioglycolic acid. A more preferred example of the spacer includes a residue obtained after ester linkage of both of a

2

steroid derivative and a polysaccharide with each of the following polycarboxylic acids such as succinic acid, citric acid, malonic acid, malic acid, tartaric acid, maleic acid, phthalic acid, methylsuccinic acid, glutaric acid, methylglutaric acid, dimethylglutaric acid, and methylethylglutaric acid. An especially preferred example of the spacer includes a residue obtained after ester linkage of both of a steroid derivative and a polysaccharide with each of the following polycarboxylic acids such as maleic acid, succinic acid, phthalic acid and glutaric acid.

In order to produce the compound obtained as a result of combination of a steroid derivative with a polysaccharide according to the present invention, there is, for example, a process as described herein − below.

About 70 mg of a steroid are added to a suitable solvent, for example, 3 ml of dimethyl sulfoxide to dissolve the steroid, about 20 mg of acid anhydride is added thereto, and the resulting mixture is reacted at 100 − 150ºC for 2 − 6 hours to obtain an esterified steroid. 50 mg of N, N' − carbonyldiimidazole are added to the solution thus obtained. The solution is agitated at room temperature for 15 minutes, followed by adding thereto 2.0 ml of triethylamine (1 mg/ml) solution in which 4 − pyrolidinopyridine is contained in 15 ml of dimethyl sulfoxide, and 350 mg of a polysaccharide having a suitable molecular weight. The resulting mixture is agitated at room temperature in shade for 72 hours. To the reaction mixture thus obtained are added 70 ml of absolute alcohol, the mixture is allowed to stand overnight under cooling, and a precipitate is obtained by filtration. The precipitate is washed with absolute alcohol, and then dried under reduced pressure so that polysaccharide derivative powder of the steroid can be obtained.

A ratio of a steroid derivative in the compound obtained as a result of combination of a steroid derivative with a polysaccharide according to the present invention is not concluded collectively, because it is significantly different dependent on a molecular weight of the polysaccharide used, but such ratio ranges generally from 0.01 to 20% by weight of the compound.

Functions

When the compound according to the present invention is administered intravenously, the compound is hydrolyzed due to the physiological condition of an organism administered or enzymes in vivo, so that a steroid derivative is gradually released to take effect of the medicine. Accordingly, the compounds according to the present invention may be called by prodrugs. When the compound according to the present invention is administered, a concentration of a steroid derivative in blood comes to be not such a high degree that side effects appear even immediately after the administration. Furthermore, since the concentration of such steroid derivative in blood is maintained at a certain value for many hours, it is not required to administer such steroid derivative frequently nor to drip intravenously the same.

Brief Description of the Drawings

Figure 1 shows chromatograms wherein some prednisolone − dextran derivative are treated with sodium hydroxide and the others are not treated with sodium hydroxide.

Figure 2 is a graphical representation showing concentrations of prednisolone in blood plasma in the case when prednisolone or a prednisolone − dextran derivative is administered intravenously to rats.

Examples

Specific examples are enumerated hereinbelow and the present invention will be described in more detail, but it is to be noted that the invention is not limited to these examples.

Example 1.

70 mg of dexamethasone are added to 3.0 ml of dimethyl sulfoxide to dissolve the former, and 22 mg of glutaric anhydride are added thereto so as to react at 120˚C for 4 hours. A temperature of the resulting solution is permitted to return to room temperature, and 50 mg of N, N' − carbonyldiimidazole are added thereto. The resulting solution is agitated at room temperature for 15 minutes, then a mixture of 2.0 ml of triethylamine (1 mg/ml) solution in which 4 − pyrolidinopyridine is contained in 15 ml of dimethyl sulfoxide and 350 mg of Dextran T − 70 is added thereto, and the solution thus obtained is agitated at room temperature in shade for 3 days. Thereafter 70 ml of absolute ethanol are added to the resulting reaction mixture to obtain a colloidal substance, the substance thus obtained is allowed to stand overnight under cooling, and a precipitate is obtained by filtration. The precipitate is washed with 10 ml of absolute ethanol two times, the precipitate so washed is obtained by filtration, and then the resulting precipitate is dried in the presence of phosphorus pentoxide under reduced pressure for 1 day, whereby a compound in which dexamethasone combines with Dextran T－70 through glutaric acid was obtained in powdery form.

Example 2.

The procedure of Example 1 is carried out by the same manner except that 70 mg of prednisolone and 20 mg of succinic anhydride are used in place of dexamethasone and gulutaric anhydride, respectively, thereby obtaining in powdery form a compound in which prednisolone combines with Dextran T－70 through succinic acid.

Example 3.

The procedure of Example 1 is carried out by the same manner except that 71 mg of hydrocortisone, 20 mg of succinic anhydride, and Dextran T－40 are used in place of dexamethasone, glutaric anhydride, and Dextran T－70, respectively, whereby a compound in which hydrocortisone combines with Dextran T－40 through succinic acid was obtained in powdery form.

Example 4.

The procedure of Example 1 is carried out by the same manner except that 70 mg of prednisolone are used in place of dexamethasone, whereby a compound in which prednisolone combines with Dextran T－70 through glutaric acid was obtained in powdery form.

Example 5.

The procedure of Example 1 is carried out by the same manner except that 70 mg of prednisolone and 22 mg of methylsuccinic anhydride are used in place of dexamethasone and glutaric anhydride, respec－ tively, whereby a compound in which prednisolone combines with Dextran T－70 through methylsuccinic acid was obtained in powdery form.

Example 6.

90 mg of prednisolone hemisuccinate are added to 3.0 ml of dimethyl sulfoxide to dissolve the former, and 50 mg of N, N'－carbonyldiimidazole are added to the solution thus obtained. The resulting solution is agitated at room temperature for 15 minutes, then a mixture of 2.0 ml of triethylamine (1 mg/ml) solution in which 4－pyrolidinopyridine is contained in 15 ml of dimethyl sulfoxide and 350 mg of Dextran T－10 is added thereto, and the solution thus obtained is agitated at room temperature under glare protection for 3 days. Thereafter 70 ml of absolute ethanol are added to the resulting reaction mixture to obtain a colloidal substance, the substance thus obtained is allowed to stand overnight under cooling, and a precipitate is obtained by filtration. The precipitate is washed with 10 ml of absolute ethanol two times, the precipitate so washed is obtained by filtration, and then the resulting precipitate is dried in the presence of phosphorus pentoxide under reduced pressure for 1 day, whereby a Dextran T－10 derivative of prednisolone hemisuccinate was obtained in powdery form.

Example 7.

The procedure of Example 6 is carried out by the same manner except that 350 mg of Dextran T－40 are used in place of Dextran T－10, whereby a Dextran T－40 of prednisolone hemisuccinate was obtained in powdery form.

Example 8.

The procedure of Example 6 is carried out by the same manner except that 350 mg of Dextran T－70 are used in place of Dextran T－10, whereby a Dextran T－70 derivative of prednisolone hemisuccinate was obtained in powdery form.

4

Example 9.

The procedure of Example 6 is carried out by the same manner except that 350 mg of Dextran T−500 are used in place of Dextran T−10, whereby a Dextran T−500 derivative of prednisolone hemisuccinate was obtained in powdery form.

Example 10.

The procedure of Example 6 is carried out by the same manner except that 350 mg of pullulan are used in place of Dextran T−10, whereby a pullulan derivative of prednisolone hemisuccinate was obtained in powdery form.

Advantages of the Invention

The advantages obtained by the present invention will be described in accordance with the following experimental examples.

Experimental Example 1: HPLC Analysis for a Dextran Derivative of Prednisolone

(1) Sample

Dextran derivatives of prednisolone prepared in Examples 2, 6, 7 and 8.

(2) Process

10 mg of a dextran derivative of prednisolone was dissolved in 5 ml of 25% acetonitrile−water, to 0.9 ml of the resulting solution was added 0.1 ml of 1N aqueous sodium hydroxide, the mixture was permitted to stand at room temperature for 1 minute, then 0.1 ml of 1N aqueous hydrochloric acid was added thereto to neutralize the same, 1.0 ml of water was further added thereto, and the resulting mixture was cooled in ice−water to obtain a sample solution for HPLC.

1 mg of prednisolone was dissolved in 25% acetonitrile−25% ethanol aqueous solution, and the resulting solution was treated by the same manner as that described above to obtain a standard sample solution.

HPLC was carried out with respect to the sample solution and the standard solution in accordance with the following conditions, whereby a prednisolone content in the prednisolone−dextran derivatives was determined.

HPLC Conditions

Column; TSK gel ODS−80TMCTR (5 μm) 4.6 mm ID x 10 cm
Mobile Phase; Acetonitrile : Water (3 : 7)
Rate of Flow; 1.5 ml/min
Detection; 254 nm

(3) Results

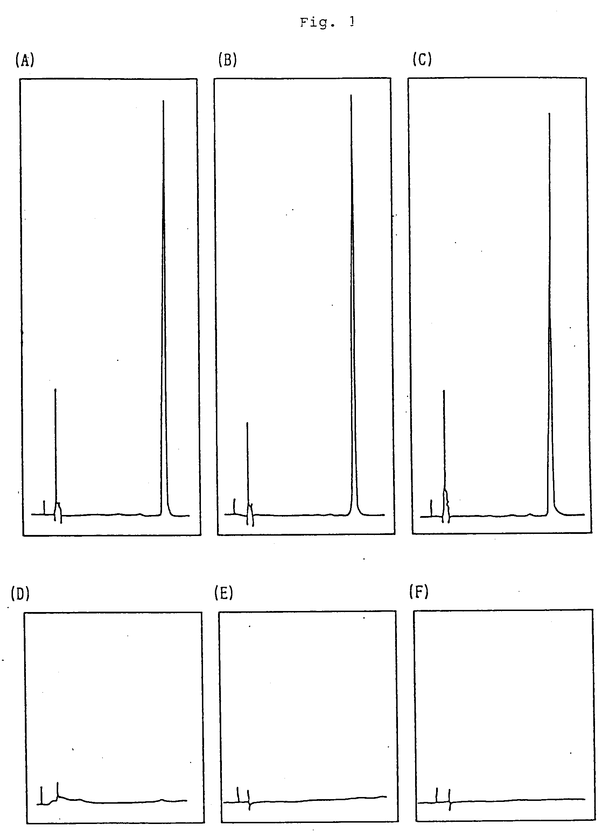

The results of HPLC analysis were shown in Figure 1 A) − F) wherein A) − C) showed chromatograms obtained as a result of treating Dextran T−10, T−40 and T−70 derivatives of prednisolone with sodium hydroxide, respectively. From the results, it is apparent that the prednisolone is liberated from these derivatives, respectively. Figure 1 D) − F) showed chromatograms obtained as a result of not treating Dextran T−10, T−40 and T−70 derivatives of prednisolone with sodium hydroxide. It is apparent that these derivatives contain scarcely free prednisolone, but the prednisolone combines chemically with the dextran through succinic acid. A ratio of combination (% by weight) of prednisolone in the prednisolone−Dextran T−10, T−40 and T−70 derivatives was shown in Table 1, respectively.

Table 1

| Combined Dextran | NMR method (%) | HPLC method (%) |
|---|---|---|
| Prednisolone + Dextran T − 10 | 11.85 | 11.05 |
| Prednisolone + Dextran T − 40 | 9.95 | 11.14 |
| Prednisolone + Dextran T − 70 | 8.87 | 9.31 |

Experimental Example 2: $^1$H − NMR Analysis for Dextran Derivatives of Prednisolone

(1) Sample

Dextran derivatives of prednisolone prepared in Examples 2, 6, 7 and 8.

(2) Process

1 − 2 mg of a dextran derivative of prednisolone was permitted to dissolve into $D_2O$ to measure $^1$H − NMR.

(3) Results

A signal derived from dextran and a characterized signal of prednisolone were confirmed.

δ = 5.1 ppm (1H, s, anomeric protons derived from dextran)

δ = 7.7 ppm (1H, s, allyl protons derived from prednisolone)

Integrals were determined with respect to the above described signals of protons, and a ratio of combination (% by weight) of prednisolone in the prednisolone − dextran derivatives was calculated in accordance with the following equation.

$$\text{Weight of prednisolone (\%)} = \frac{\text{Molecular weight of prednisolone} \times \dfrac{\text{Integral of allyl protons of prednisolone}}{\text{Integral of anomeric protons of dextran}}}{\text{One unit of polysaccharide in terms of molecular weight} \times \text{Molecular weight of prednisolone} \times \dfrac{\text{Integral of allyl protons of prednisolone}}{\text{Integral of anomeric protons of dextran}}}$$

The results were shown in Table 1.

Experimental Example 3.

A change of the concentration of prednisolone − dextran derivatives in blood after administering intravenously the same

(1) Sample

10% ethanol physiologic saline is added to prednisolone and the prednisolone − Dextran T − 70 derivatives obtained in Examples 2 and 8 to dissolve the latter, respectively, whereby concentrations of the prednisolone and the prednisolone − Dextran T − 70 derivatives are adjusted so as to be 1.50 mg/ml and 17.05 mg/ml, respectively, to obtain administering solution samples for rats.

(2) Process

Each of the administering solution samples of the prednisolone or the prednisolone − Dextran T − 70 derivatives is administered intravenously to each rat through its femoral region in such that a concentration of the former sample is 1.50 mg/kg and that of the latter is 17.05 mg/kg, respectively. After the elapse of 5 minutes, 15 minutes, 30 minutes, 60 minutes, 2 hours, 4 hours and 6 hours, 0.25 ml of blood is collected into a syringe barrel treated with heparin through the jugular vein of a rat, respectively, and the collected blood in the syringe barrel is subjected to centrifugal separation at 3,000 rpm for 10 minutes to obtain blood plasma.

To 0.1 ml of each blood plasma taken out from the syringe barrel are added 0.2 ml of water, and further 2 ml of acetonitrile, respectively, the resulting mixture is subjected to centrifugal separation at 3,000 rpm for 10 minutes, and then the supernatant liquid is collected. To the supernatant liquid are further added 5 ml of n − hexane, the resulting solution is agitated, then the solution is subjected to centrifugal separation at 3,000 rpm for 5 minutes to remove a n − hexane layer of the upper layer and distill away the lower layer solvent, 0.2 ml of methanol − water (1 : 1) solution is added to the resulting substance and is agitated. The solution thus obtained is subjected to centrifugal separation at 3,000 rpm for 10 minutes, HPLC is carried out with respect to the resulting supernatant in accordance with the conditions in Experimental Example 1 to examine a change in the concentration of prednisolone in the blood plasma.

(3) Results

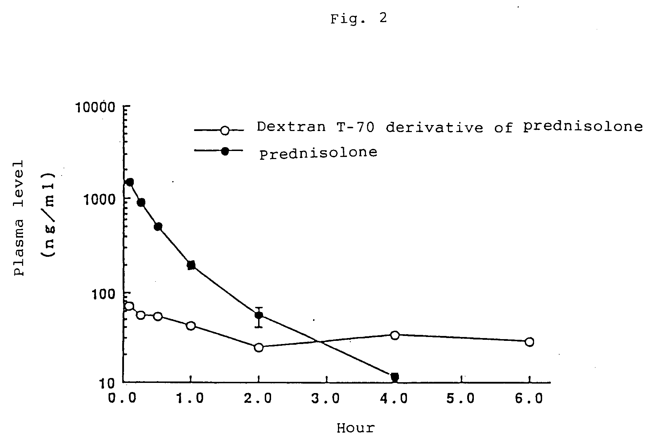

Changes in concentrations of prednisolone in blood plasma after administering intravenously pred − nisolone or prednisolone − Dextran T − 70 derivatives to rats were shown in Figure 2.

From these results, it is apparent that an extremely high concentration in blood plasma is observed at an early stage of administration of prednisolone and it disappears rapidly from the blood, and on the other hand, a concentration in blood plasma does not increase even at an early stage of administration of prednisolone − Dextran T − 70 derivative and remarkable decrease of the concentration in blood plasma is not observed even 6 hours after the administration.

**Claims**

1. A compound comprising a steroid derivative and a polysaccharide bonded therewith either directly or through a spacer.

2. A compound as claimed in claim 1 wherein said steroid derivative is ones each having carboxyl group and/or hydroxyl group.

3. A compound as claimed in claim 1 wherein said polysaccharide is dextran, dextrin, starch, partially hydrolyzed starch, and pullulan.

4. A compound as claimed in claim 1 wherein said spacer is the residue of a polycarboxylic acid obtained after esterification of a steroid derivative with a polysaccharide.

5. A compound as claimed in claim 1 wherein said spacer is the residue of succinic acid, citric acid, malonic acid, malic acid, tartaric acid, maleic acid, phthalic acid, methylsuccinic acid, glutaric acid, methylglutaric acid, dimethylglutaric acid or methylethylglutaric acid subsequent to esterification of the steroid derivative with a polysaccharide.

6. A compound as claimed in claim 1 wherein said steroid derivative is hydrocortisone, prednisolone, dexamethasone, triamcinolone, aldosterone, corticosterone, cloprednol, deflzacort, prednisone, methyl − prednisolone, betamethasone, paramethasone, $9\alpha$ − fluorocortisol, or a derivative thereof.

Fig. 1

(A)

(B)

(C)

(D)

(E)

(F)

Fig. 2

# INTERNATIONAL SEARCH REPORT

International Application No PCT/JP92/00638

**I. CLASSIFICATION OF SUBJECT MATTER** (if several classification symbols apply, indicate all) [6]

According to International Patent Classification (IPC) or to both National Classification and IPC

Int. Cl$^5$  C07J17/00

**II. FIELDS SEARCHED**

Minimum Documentation Searched [7]

| Classification System | Classification Symbols |
|---|---|
| IPC | C07J1/00-53/00, A6K31/705, 715, 31/565-585, 9/08-133 |

Documentation Searched other than Minimum Documentation
to the Extent that such Documents are Included In the Fields Searched [8]

**III. DOCUMENTS CONSIDERED TO BE RELEVANT** [9]

| Category [*] | Citation of Document, [11] with Indication, where appropriate, of the relevant passages [12] | Relevant to Claim No. [13] |
|---|---|---|
| Y | JP, A, 62-46555 (Ichimaru Farukos K.K.), October 2, 1987 (02. 10. 87), line 8, left column to line 31, right column, page 2, (Family: none) | 1-6 |
| A | JP, A, 55-22616 (Tokyo Tanabe Co., Ltd.), February 18, 1980 (18. 02. 80), lower left column, page 2, (Family: none) | 1-6 |
| A | JP, A, 58-83699 (Richter Gedeon Vegyeszeti Gyar Rt.), May 19, 1983 (19. 05. 83), line 4, upper left column to line 3, upper right column, page 4, (DE, A1, 3238800) | 1-6 |
| A | JP, A, 62-123196 (Nisshin Flour Milling Co., Ltd.), June 4, 1987 (06. 06. 87), line 13, lower right column, page 1 to line 20, upper right column, page 2 (Family: none) | 1-6 |

\* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 15, 1992 (15. 07. 92) | August 4, 1992 (04. 08. 92) |

| International Searching Authority | Signature of Authorized Officer |
|---|---|
| Japanese Patent Office | |

Form PCT/ISA/210 (second sheet) (January 1985)